# EUROPEAN PATENT SPECIFICATION

(11) **EP 1 450 721 B1**
(45) Date of publication and mention of the grant of the patent: **03.01.2007**
(21) Application number: 02766443.2
(22) Date of filing: 03.10.2002
(51) Int. Cl.: A61C 7/14, A61C 7/00

(54) **METHOD FOR THE ENHANCEMENT OF ORTHODONTIC TREATMENTS**
VERFAHREN ZUM VERBESSERN VON ORTHODONTISCHE BEHANDLUNGEN
METHODE D'AMELIORATION DE TRAITEMENTS ORTHODONTIQUES

(30) Priority: 27.11.2001 US 995456
(43) Date of publication of application: 01.09.2004
(73) Proprietor: 3M Innovative Properties Company, St. Paul, MN 55133-3427 (US)
(72) Inventor: MCLAUGHLIN, Richard, P., Saint Paul, MN 55133-3427 (US)
(74) Representative: Vossius & Partner
(86) International application number: PCT/US2002/031237
(87) International publication number: WO 2003/045267

(56) References cited:
- US-A- 5 683 243
- US-A- 5 951 498

## Description

### Technical Field of the Invention

The present invention relates to a method which is useful in orthodontically treating patients.

### Background of the Invention

Orthodontics is a branch of dentistry that involves the movement of malpositioned teeth to orthodontically correct positions. Before prescribing an orthodontic treatment, X-rays and photographs of the patient's teeth and jaw structure are usually taken. Also, a mold of the patient's teeth is typically made. This mold along with the X-rays and photographs provide a model of the positions of the patient's teeth and dental arches prior to treatment.

The orthodontist also frequently relies on a post treatment model of the desired positions of the patient's teeth and dental arches. This post-treatment model has typically been a mental model formulated in the mind of the orthodontist based on the orthodontist's experience and skill. However, computer programs are also known to assist the orthodontist in the development of a computerized post-treatment model. The orthodontist then devises a treatment strategy to move the patient's teeth and/or dental arches from their positions as represented by the pre-treatment model to the desired positions as represented by the post-treatment model.

Cephalometric analyses are also an important part of orthodontic diagnosis and treatment planning. Most of these cephalometric analyses measure maxillary (upper jaw) and mandibular (lower jaw) skeletal relationships in the vertical and horizontal planes, as well as the positions and angulations of the incisors. Few cephalometric analyses provide information about the direction and amount of dental movements required in the maxillary and mandibular arches.

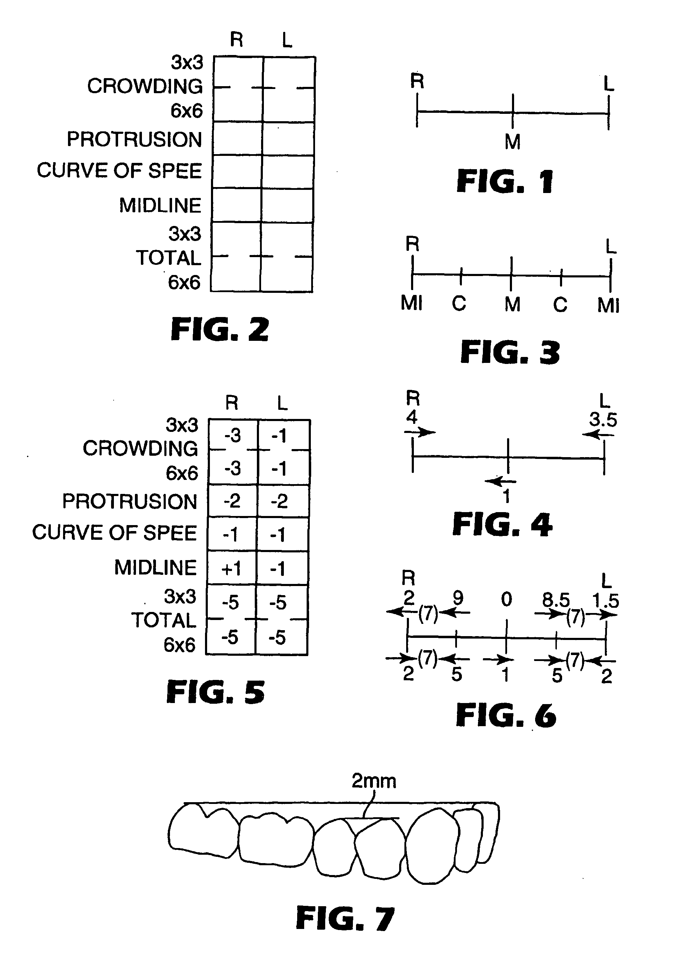

It is helpful to have additional dental information to assist in the diagnosis and treatment planning process. This information should include the initial position and desired movement of the first molars, canines, and dental midlines. The charts shown in Figures 1, 2, and 3 have been used in the past to provide some of this additional information. Figures 4, 5, and 6 show these charts with an exemplary set of patient data.

The chart of Figure 1 is used to record the patient's initial midline and first molar relationships on the right and left sides. The exemplary data in Figure 4 indicate that the upper dental midline of the patient is symmetrical and that the lower dental midline deviates from the upper dental midline 1 mm to the right. The molar relationship data of Figure 4 indicate a 4 mm Class II deviation between the upper and lower first molars of the patient on the right side and a 3.5 mm Class II deviation between the upper and lower first molars of the patient on the left side.

The chart of Figure 2 is used to record the lower arch discrepancy. Four primary arch factors are used. The space required for relief of crowding between the canine and the midline (3x3) and the space required for relief of crowding between the first molar to the midline (6x6) are recorded for each side, right and left. The space required to correct protrusion or intrusion of the lower incisors is also recorded as is the space required to level the curve of Spee. The space required for correction of the midline is further recorded. Finally, these space requirements are totaled.

It has been suggested that four secondary factors can also be listed below the chart of Figure 2. These four secondary factors include additional space from interproximal enamel reduction, additional space from uprighting or distal movement of the lower arch first molars, additional space from buccal uprighting of the lower canines and the lower posterior teeth, and additional space from leeway space or "E" space. Buccal uprighting is the widening of the lower arch (teeth, not bone). Leeway space is the size difference between primary canines, first molars, and second molars, and the corresponding permanent canines, first premolars, and second premolars.

The exemplary data in Figure 5 indicate that the lower arch has 3 mm of crowding on the right side, all of which is mesial to the lower right canine. In other words, there is 3 mm of crowding between the lower right canine and the midline. The chart in Figure 5 indicates that, on the lower right side, the crowding between the first molar and the midline is also 3 mm, meaning that all of the crowding on the lower right side is between the canine and the midline.

Similarly, the lower arch has only 1 mm of crowding on the left side, all of which is mesial to the lower left canine. The chart in Figure 5 indicates that, on the lower left side, the crowding between the first molar and the midline is also 1 mm, meaning that all of the crowding on the lower left side is between the canine and the midline.

As indicated by the chart of Figure 5, 2 mm of space is required on the lower right and left sides in order to correct for the protrusion (or intrusion) of the lower incisors. Also, 1 mm of space is required on the lower right and lefts sides in order to correct the curve of Spee. The curve of Spee is shown in Figure 7. The depth of the curve of Spee is measured at its deepest point. Ideally, the curve of Spee should be level, i.e., having zero depth. However, in the example of Figures 5 and 7, it is assumed that the curve of Spee is 2 mm deep on each side. The curve of Spee depth is measured from a line extending from the distal cusps of the second molars to the incisal edges of the central incisors. It is generally believed that leveling a curve of Spee that is 2 mm deep requires 1 mm of space. Thus, the exemplary data of Figure 5 indicates that 1 mm of space is needed in order to level the curve of Spee.

Further, as discussed above in connection with Figure 4, the lower midline is deviated 1 mm to the right. Because the lower midline is deviated 1 mm to the right, correction of the lower midline requires 1 mm of space on the left side so that the midline can be moved 1 mm to the left. On the other hand, movement of the midline 1 mm to the left creates 1 mm of the space on the right side. Accordingly, the 1 mm of created space on the right side is shown as +1 in the chart of Figure 5, and the 1 mm of required space on the left side is shown as -1 in the chart of Figure 5.

Finally, the space requirements are totaled in each region (3x3 and 6x6). Thus, on the lower right side, 5 mm of space is needed between the canine and the midline to alleviate the crowding, to retract the lower incisor protrusion, and to level the curve of Spee. Based on the data in the chart of Figure 5, this space requirement is 6 mm (3+2+1). However, because 1 mm of space is created on the right by moving the midline 1 mm to the left, the total space requirement is recorded in the chart of Figure 5 as -5, where the minus sign indicates required space rather than created space. Also, because all of the required space is in the 3x3 region, the same total space requirement applies to the 6x6 region.

Similarly, on the lower left side, 5 mm of space is needed between the canine and the midline to alleviate the crowding, to retract the lower incisor protrusion, to level the curve of Spee, and to correct the midline. Based on the data in the chart of Figure 5, this space requirement is 5 mm (1+2+1+1). Again, the minus sign in the chart of Figure 5 indicates required space rather than created space. Also, because all of the needed space is in the 3x3 region, the same requirement applies to the 6x6 region.

An upper arch discrepancy chart would not ordinarily be done for the patient because it is generally assumed that correct positioning of the lower teeth will align the lower teeth with the upper teeth. However, an upper arch discrepancy chart can be useful to the orthodontist such as in surgical cases.

Once the patient data have been inserted into the charts of Figures 1 and 2 as shown by the charts of Figures 4 and 5, anticipated treatment changes can be recorded in the chart of Figure 3. The anticipated treatment based upon the exemplary data shown in Figures 4 and 5 is shown in the chart of Figure 6. The anticipated treatment recognizes that, if extractions are not made, the malpositioned lower incisors could not be correctly positioned and, in fact, the positions of the incisors would most probably be made worse. Because these positions of the lower incisors are considered to be unacceptable, extractions of the upper and lower, right and left first premolars are included in the anticipated treatment. Further, because of these extractions, it becomes unnecessary to consider interproximal reduction, uprighting of the lower arch first molars, and/or buccal uprighting of the lower canines and the lower posterior teeth.

The extraction of the four first premolars normally creates 7 mm of space in each quadrant (upper right, upper left, lower right, and lower left). These spaces are recorded in the chart of Figure 6 as (7) in each quadrant. Because the total lower arch discrepancy from midline to canine on each side is 5 mm as shown by the chart in Figure 5, the lower left and right canines can be retracted into the extraction sites to correct this discrepancy. These 5 mm movements are recorded in the chart of Figure 6 as 5 with arrows pointing distally in the lower right and left quadrants.

Because there is an extra 2 mm of space from the extractions after the canines are moved distally by 5 mm, the lower molars are moved 2 mm mesially on the lower right and left sides in order to close the extra 2 mm of space in each of the lower extraction sites. These 2 mm movements are recorded in the chart of Figure 6 as 2 with arrows pointing mesially in the lower right and left quadrants.

As indicated in the charts of Figures 4 and 5, the lower midline is deviated 1 mm to the right and, therefore, the lower midline is moved 1 mm to the left. This 1 mm movement is recorded in the chart of Figure 6 as 1 with an arrow pointing to the left in the lower jaw.

As shown by the chart of Figure 6, the total upper arch discrepancy from midline to canine on the right side is 9 mm and the total upper arch discrepancy from midline to canine on the left side is 8.5 mm. Correction of these discrepancies requires the upper right canine to be retracted by 9 mm into the upper right extraction site and the upper left canine to be retracted by 8.5 mm into the upper left extraction site. These 9 mm and 8.5 mm movements are recorded in the chart of Figure 6 as 9 and 8.5, respectively, with corresponding arrows pointing distally in the upper right and left quadrants.

Because an additional 2 mm of space is still required in the upper right quadrant, the upper right molars are moved 2 mm distally in order to create the additional 2 mm of space. Similarly, because an additional 1.5 mm of space is still required in the upper left quadrant, the upper left molars are moved 1.5 mm distally in order to create the additional 1.5 mm of space. These 2 mm and 1.5 mm movements are recorded in the chart of Figure 6 as 2 with corresponding arrows pointing distally in the upper right and left quadrants.

As indicated in the charts of Figures 4 and 5, no movement of the upper midline is required.

These charts provide useful analytical tools to the treating orthodontist. However, there is additional information that can be provided to the treating orthodontist in a form that helps the orthodontist to develop better treatment strategies. The present invention is directed to an arrangement that includes this additional information.

Moreover, United States Patent No. 5,683,243 discloses that custom orthodontic appliances can be automatically fabricated by deriving final positions of the teeth of a patient from digitized information of the anatomical shape of the patient's mouth, and by using the digitized shape information and the derived tooth final positions in the design of the appliances. The digitized information is generated from measurements of the mouth of the patient, and includes information of the shapes of the individual teeth of the patient and of the patient's lower jaw. The final tooth positions include an archform defined by the shape of the lower jaw, and the appliance is configured in accordance with this archform. An archwire forming machine reads input data related to the shape of the patient's jaw and teeth, derives the tooth final positions as well as archwire and bracket designs that will move the teeth to the calculated final positions, and generates code to produce the archwire in accordance with the design. A bracket fabrication machine fabricates the brackets based on the final tooth position calculations and digitized tooth shape data, and determines positions on the teeth to receive archwires.

### Summary of the Invention

The present invention is defined by the method according to the features of the claims.

In accordance with a preferred embodiment, the method comprises the following: entering first crowding/spacing data in first and second tables, wherein the first table relates only to cuspid to midline regions of a patient's jaw, wherein the second table relates to second molar to midline regions of the patient's jaw, and wherein the first crowding/spacing data relates to the right and left cuspid to midline regions of the patient's jaw; entering second crowding/spacing data in the second table but not the first table, wherein the second crowding/spacing data relates to bicuspid regions of the patient's jaw; entering third crowding/spacing data in the second table but not the first table, wherein the third crowding/spacing data relates to molar regions of the patient's jaw; entering curve of Spee spacing data in the first and second tables, wherein the curve of Spee spacing data relates to space required to correct a curve of Spee of the patient's jaw; entering midline spacing data in the first and second tables, wherein the midline spacing data relates to space created and required to move a midline of teeth in the patient's jaw; entering incisor position data in the first and second tables, wherein the incisor position data relates to space required to correct positions of incisors in the patient's jaw; creating for the first table but not the second table a first total by summing the first crowding/spacing data, the curve of Spee spacing data, the midline spacing data, and the incisor position data; and, creating for the second table but not the first table a second total by summing the first crowding/spacing data, the second crowding/spacing data, the third crowding/spacing data, the curve of Spee spacing data, the midline spacing data, and the incisor position data.

In accordance with another preferred embodiment, the method comprises the following: entering first crowding/spacing data in first and second tables, wherein the first table contains data related only to cuspid to midline regions of a patient's jaw, wherein the second table relates to second molar to midline regions of the patient's jaw and includes the data related to the cuspid to midline regions of the patient's jaw, and wherein the first crowding/spacing data relates to cuspid to midline regions of the patient's jaw; entering second crowding/spacing data in the second table, wherein the second crowding/spacing data relates to bicuspid regions of the patient's jaw; entering third crowding/spacing data in the second table, wherein the third crowding/spacing data relates to molar regions of the patient's jaw; entering curve of Spee spacing data in the first and second tables, wherein the curve of Spee spacing data relates to space required to correct a curve of Spee of the patient's jaw; entering midline spacing data in the first and second tables, wherein the midline spacing data relates to space created and required to move a midline of teeth in the patient's jaw; entering incisor position data in the first and second tables, wherein the incisor position data relates to space required to correct positions of incisors in the patient's jaw; summing the first crowding/spacing data, the curve of Spee spacing data, the midline spacing data, and the incisor position data to create a first total and entering the first total in the first table as a first initial discrepancy; summing the first crowding/spacing data, the second crowding/spacing data, the third crowding/spacing data, the curve of Spee spacing data, the midline spacing data, and the incisor position data to create a second total and entering the second total in the second table as a second initial discrepancy; entering other created space in the first and second tables; summing the first total and the other created space to create a third total and entering the third total in the first table as a first remaining discrepancy; and, summing the second total and the other created space to create a fourth total and entering the fourth total in the second table as a second remaining discrepancy.

In accordance with yet another preferred embodiment, the method to comprises the following: entering midline and molar relationships into a midline chart; entering first, second, third, fourth, fifth, and sixth crowding/spacing data into a discrepancy chart having first and second tables, wherein the first table contains data related only to cuspid to midline regions of a patient's jaw, wherein the second table relates to second molar to midline regions of the patient's jaw and includes the cuspid to midline regions of the patient's jaw, wherein the first crowding/spacing data relates to cuspid to midline regions of the patient's jaw, wherein the second crowding/spacing data relates to bicuspid regions of the patient's jaw, wherein the third crowding/spacing data relates to molar regions of the patient's jaw, wherein the fourth crowding/spacing data relates to a curve of Spee, wherein the fifth crowding/spacing data relates to midline position, and wherein the sixth crowding/spacing data relates to incisor position; and, entering data from the first and second tables into an anticipated treatment chart.

### Brief Description of the Drawing

These and other features and advantages will become more apparent from a detailed consideration of the invention when taken in conjunction with the drawings in which:
Figure 1 illustrates a known blank chart that may be used to record a patient's initial midline and first molar relationships;
Figure 2 illustrates a known blank chart that can be used to record the lower arch discrepancy;
Figure 3 illustrates a known blank chart that can be used to record anticipated treatment changes for a patient;
Figure 4 illustrates exemplary data for a patient as recorded in the chart of Figure 1;
Figure 5 illustrates exemplary data for the patient as recorded in the chart of Figure 2;
Figure 6 illustrates anticipated treatment changes for the patient as recorded in the chart of Figure 3 and as based on the exemplary data shown in Figures 4 and 5;
Figure 7 is a diagram useful in understanding the curve of Spee;

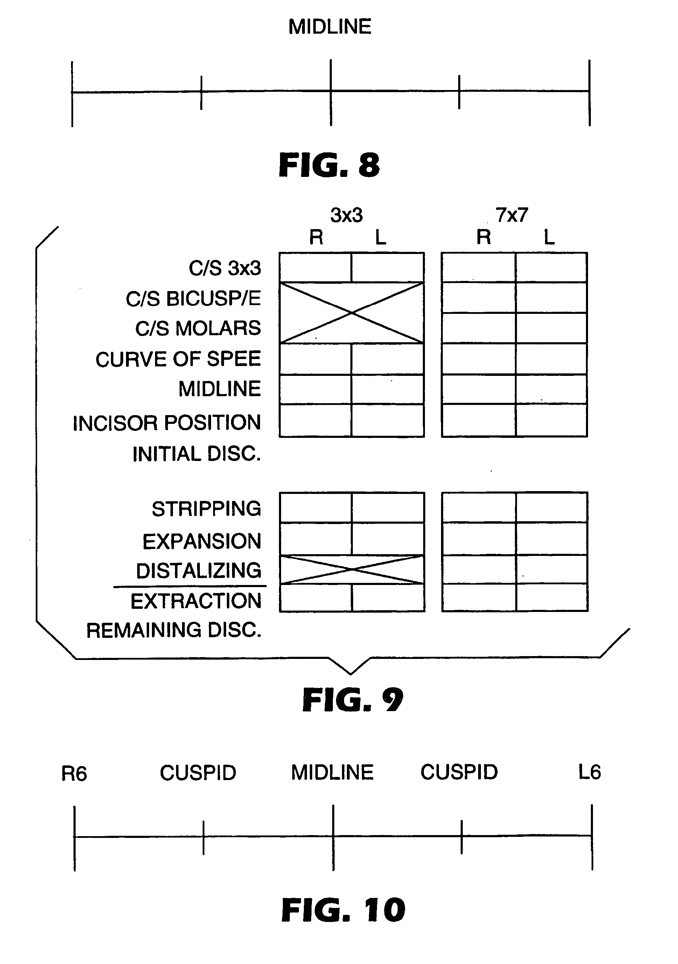

Figure 8 illustrates a blank chart that may be used in accordance with the present invention to record a patient's initial midline and first molar relationships;
Figure 9 illustrates a blank chart that may be used in accordance with the present invention to record the lower arch discrepancy;
Figure 10 illustrates a blank chart that may be used in accordance with the present invention to record anticipated treatment changes for a patient;

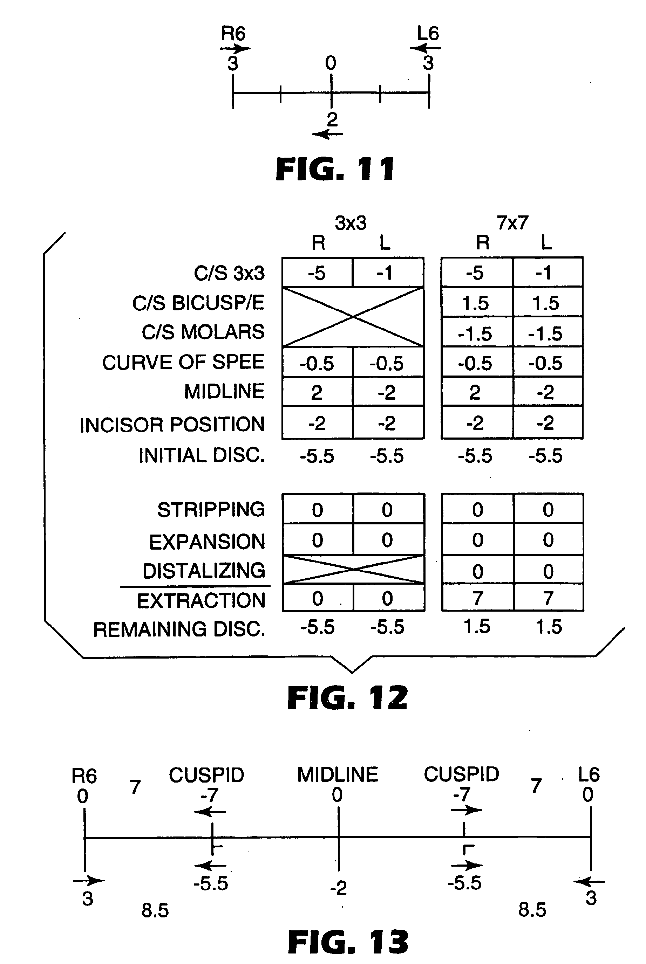

Figure 11 illustrates exemplary data for a patient as recorded in the chart of Figure 8;
Figure 12 illustrates exemplary data for the patient as recorded in the chart of Figure 9;
Figure 13 illustrates anticipated treatment changes for the patient as recorded in the chart of Figure 10 and is based on the exemplary data shown in Figures 11 and 12; and

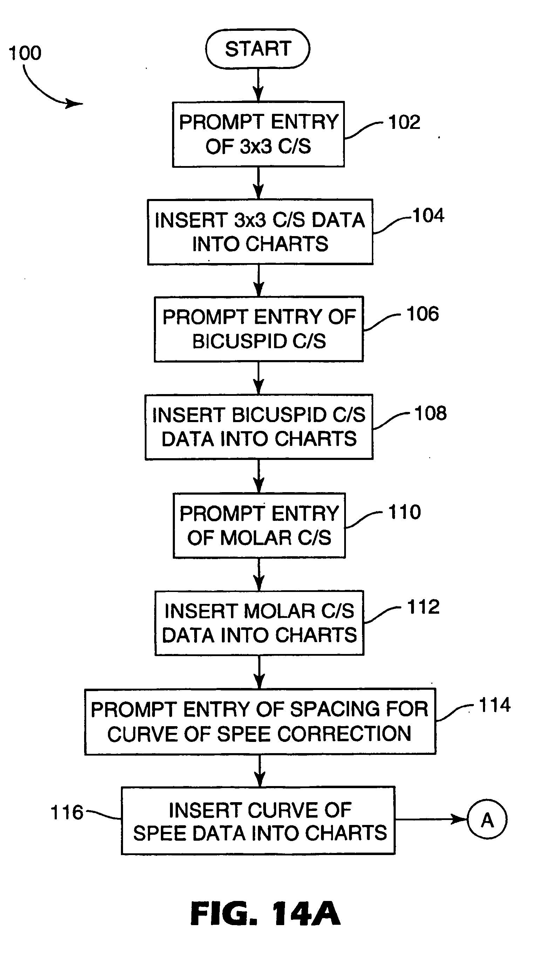

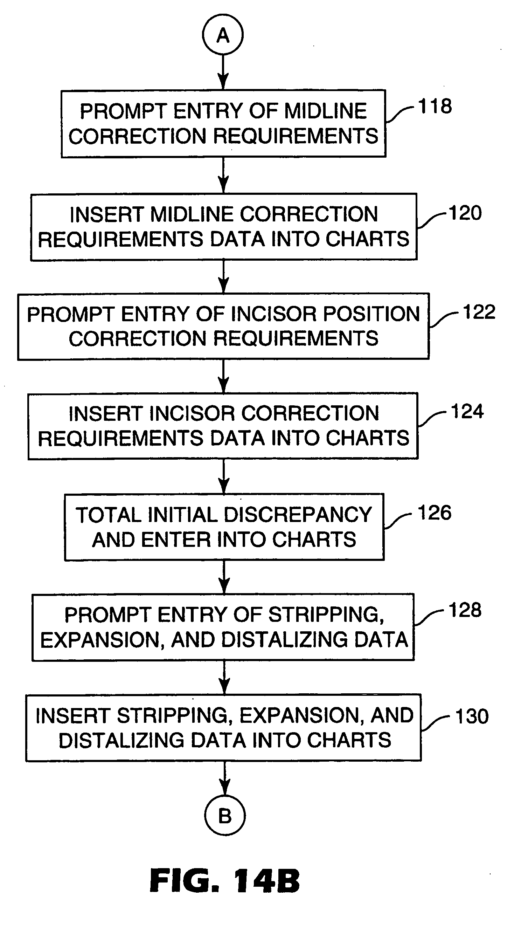

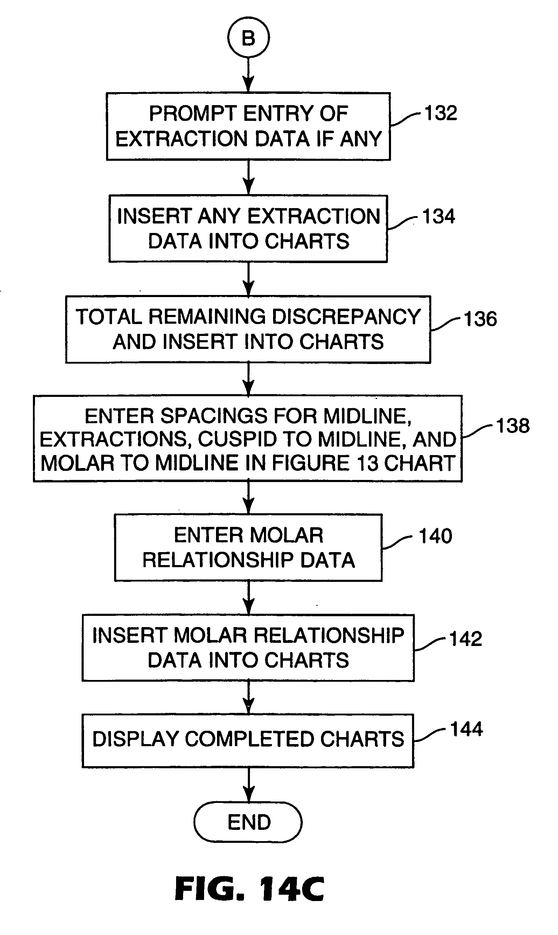

Figures 14A-14C illustrate a flow chart representing a program that may be implemented by a computer in order to carry out the present invention.

### Detailed Description

The chart of Figure 8 is used to record the patient's initial midline and first molar relationships on the right and left sides. The exemplary data in Figure 11 indicate that the upper dental midline of the patient is symmetrical and that the lower dental midline deviates from the upper dental midline by 2 mm to the right. The molar relationship data of Figure 11 indicate a 3 mm Class II deviation between the upper and lower first molars of the patient on the right and left side. Any overjet, overbite, and crossbite of a patient may also be recorded under the chart of Figure 8.

A chart having 3x3 and 7x7 tables is illustrated in Figure 9 and is used to record the lower arch discrepancy. The 3x3 chart covers the regions between the right canine and the midline and between the left canine and the midline. The 7x7 chart covers the regions between the right second molar and the midline and between the left second molar and the midline. The spaces required for relief of crowding between the right canine and the midline and between the left canine and the midline (3x3) are recorded in both the 3x3 table and the 7x7 table. The spaces required for the relief of crowding of the right premolars (bicuspids) and the left premolars are recorded only in the 7x7 table. The spaces required for the relief of crowding of the right molars and the left molars are also recorded only in the 7x7 table. Thus, the spaces required for relief of crowding of the premolars and molars are not recorded in the 3x3 table because the 3x3 table does not cover these regions of the arch. The space required to level the curve of Spee is also recorded in both of the tables of Figure 9. The space required to correct protrusion or intrusion of the lower incisors is further recorded in both of the tables of Figure 9. These discrepancies are totaled and the totals are recorded in both tables as initial discrepancies.

As described above, the chart of Figure 9 is used to enter space requirements to alleviate crowding, to permit correction of the curve of Spee, and to permit correction of incisor positions. However, space may also exist or be created on one or both sides of the jaw. For example, when the lower midline is moved, space is created on one side of the jaw, and this created space is recorded in the chart of Figure 9 as shown by the data in Figure 12. Also, it is possible that, instead of crowding, space may exist in the canine to midline, premolar, and/or molar regions of the arch. If so, these existing spaces would be entered into the chart of Figure 9 as positive numbers.

In addition, other space can be created to meet the space requirements, and the chart of Figure 9 allows entries of such other created space. Thus, as shown in Figure 9, the chart includes entries for interproximal reduction (stripping of enamel), for lower arch expansion (widening), and for distalization of lower molars (movement of the lower molars toward the back of the jaw), and for extractions. The differences between the total created spaces and the total space requirements are entered into both tables of the chart of Figure 9 as remaining discrepancies.

The exemplary data in the C/S 3x3 row of the 3x3 table of Figure 12 indicate that the lower arch has 5 mm of crowding on the right side. This crowding is mesial to the lower right canine. This 5 mm of crowding between the lower right canine and the midline is also entered into the same row of the 7x7 table of Figure 12. Similarly, the lower arch has only 1 mm of crowding on the left side. This crowding is likewise mesial to the lower left canine. This 1 mm of crowding between the lower right canine and the midline is entered into the C/S 3x3 row of the 3x3 and 7x7 tables of Figure 12.

The exemplary data in the C/S Bicuspid/E row of the 7x7 table shown in Figure 12 indicate that the lower arch has 1.5 mm of available space in the bicuspid region of the lower right quadrant. This 1.5 mm of available space is entered as a positive number into the 7x7 table of Figure 12. Similarly, the exemplary data indicate that the lower arch has 1.5 mm of available space in the bicuspid region of the lower left quadrant. This 1.5 mm of available space is also entered as a positive number into the 7x7 table of Figure 12. These available spaces in the premolar regions are not entered into the 3x3 table because they are outside of the coverage of the 3x3 table.

The exemplary data in the C/S Molars row of the 7x7 table of Figure 12 further indicate that the lower arch requires 1.5 mm of space in the molar region of the lower right quadrant to alleviate crowding. This 1.5 mm of required space is entered as a negative number into the 7x7 table of Figure 12. Similarly, the exemplary data indicate that the lower arch requires 1.5 mm of space in the molar region of the lower left quadrant to alleviate crowding. This 1.5 mm of required space is also entered as a negative number into the 7x7 table of Figure 12. These required spaces in the molar regions are not entered into the 3x3 table because they are outside of the coverage of the 3x3 table.

As indicated by the Curve of Spee row in the chart of Figure 12, 0.5 mm of space is required on the lower right and lefts sides in order to correct the curve of Spee. This needed space is entered into both the 3x3 and the 7x7 tables of Figure 12. Further, as discussed above in connection with Figure 11, the lower midline is dentally deviated 2 mm to the right. Because the lower midline is dentally deviated 2 mm to the right, correction of the lower midline requires 2 mm of space on the left side so that the midline can be moved 2 mm to the left. As a consequence of moving the midline 2 mm to the left, 2 mm of the space are created on the right side. Accordingly, the 2 mm of created space on the right side is shown as a positive number in the Midline row of the chart of Figure 12, and the 2 mm of required space on the left side is shown as a negative number in Midline row of the chart of Figure 12. Finally, 2 mm of space is required on the lower right and lefts sides in order to correct the positions of the lower incisors. This 2 mm of required space is entered as a negative number into the Incisor Pos. row of the 3x3 and 7x7 tables of Figure 12.

These space requirements are totaled in each region (3x3 and 7x7) as an initial discrepancy. Thus, in the 3x3 table of Figure 12, 7.5 mm of space is needed between the canine and the midline on the lower right side to alleviate crowding, to correct the positions of the lower incisors, and to level the curve of Spee. However, because 2 mm of space is created when the lower midline is moved to the left, the total space requirement on the right side is 5.5 mm, which is entered as an initial discrepancy in the R column of the 3x3 table of Figure 12. Similarly, on the lower left side, 5.5 mm of space is needed to alleviate the crowding between the canine and the midline, to correct the positions of the lower incisors, to level the curve of Spee, and to correct the midline. This total space requirement is entered as an initial discrepancy in the L column of the 3x3 table of Figure 12.

In the 7x7 table of Figure 12, 9 mm of space is needed on the lower right side to alleviate crowding, to correct the positions of the lower incisors, and to level the curve of Spee. However, because 2 mm of space is created when the lower midline is moved to the left, and because 1.5 mm of space is available in the bicuspid region, the total space requirement is 5.5 mm and is entered as an initial discrepancy in the R column of the 7x7 table shown in Figure 12. Similarly, on the lower left side, 7 mm of space is needed to alleviate crowding, to correct the positions of the lower incisors, to level the curve of Spee, and to correct the midline. However, because 1.5 mm of space is available in the bicuspid region, the total space requirement is 5.5 mm and is entered as an initial discrepancy in the L column of the 7x7 table shown in Figure 12.

As the exemplary data in the 3x3 and 7x7 tables of Figure 12 indicate, spaces created by interproximal reduction, lower arch expansion, and distalization are not anticipated. However, 7 mm of space is to be created due to extractions in the molar or premolar region. Accordingly, although the remaining discrepancy in the 3x3 region on the right and left lower quadrants is the same as the initial discrepancy, the remaining discrepancy in the 7x7 region on the right and left lower quadrants is 1.5 mm (a positive number) indicating that the 7 mm of space created by the extractions more than makes up for the required 5.5 mm required spaces.

Charts similar to that shown in Figure 12 can be completed for the upper arch such as in surgical cases.

Once the patient data have been inserted into the charts of Figures 8 and 9 as shown by the charts of Figures 11 and 12, anticipated treatment changes can be recorded in the chart of Figure 10. This recorded data is shown in Figure 13, which is a reproduction of Figure 10 but containing the pertinent data relating to the anticipated treatment changes. The anticipated treatment based upon the exemplary data shown in Figures 11 and 12 includes extractions in the upper and lower, right and left quadrants. These extractions create 7 mm of space in the upper right and left quadrants and 7 mm of space in the lower right and left quadrants. The upper extractions are recorded in the chart of Figure 13 as (7) in the upper right and left quadrants. The lower extractions are also recorded in the chart of Figure 13. However, because the lower arch also gains 1.5 mm of leeway (E) space in the lower right and left bicuspid areas, the space created by the lower extractions (7) are added to the leeway space (1.5) in the region of the lower bicuspids and the total is recorded as (8.5) in the lower right and left quadrants.

The 10.5 mm of space created in the lower right quadrant (i.e., 7 mm from the extraction + the 2 mm resulting from moving the midline to the left + 1.5 mm of leeway space in the bicuspid region) permits the teeth in the lower right quadrant to be correctly positioned. This correction in the lower right quadrant uses 5 mm of space to relieve crowding between the canine and the midline, uses 0.5 mm of space to level the curve of Spee, uses 1.5 mm of space to relieve crowding in the molar region, and uses 2 mm of space for incisor position correction. This correction leaves 1.5 mm of unused space. As a result, the rear molars in the lower right quadrant are moved forward (mesially) to take up this unused space.

The entries into the chart of Figure 13 show the 2 mm movement of the midline and the net 5.5 mm of distal movement of the teeth between the canine and the midline in the lower right quadrant. The chart of Figure 13 also shows the 8.5 mm of available space in the lower right quadrant. The difference (3 mm) between the net 5.5 mm of distal tooth movement and the 8.5 mm of available space is recorded in the chart of Figure 13 as a net of 3 mm mesially in the molar region of the lower right quadrant.

The 10 mm of space created in the lower left quadrant (i.e., 7 mm from the extraction + 1.5 mm of leeway space in the bicuspid region) permits the teeth in the lower left quadrant to be correctly positioned. This correction in the lower right quadrant uses 1 mm of space to relieve crowding between the canine and the midline, uses 0.5 mm of space to level the curve of Spee, uses 1.5 mm of space to relieve crowding in the molar region, uses 2 mm of space for midline correction, and uses 2 mm of space for incisor position correction. This correction leaves 1.5 mm of unused space. As a result, the rear molars in the lower left quadrant are moved forward to take up this unused space.

The entries into the chart of Figure 13 show the 2 mm movement of the midline and the net 5.5 mm of movement of the teeth between the canine and the midline in the lower left quadrant. The chart of Figure 13 also shows the 8.5 mm of available space in the lower left quadrant. The difference (3 mm) between the net 5.5 mm of distal tooth movement and the 8.5 mm of available space is recorded in the chart of Figure 13 as a net of 3 mm mesially in the molar region of the lower left quadrant.

The 7 mm of space created in the upper right quadrant from the extraction permits the teeth in the upper right quadrant to be correctly positioned. This correction in the upper right quadrant uses all 7 mm of space to relieve crowding between the canine and the midline. Similarly, the 7 mm of space created in the upper left quadrant from the extraction permits the teeth in the upper left quadrant to be correctly positioned. This correction in the upper left quadrant uses all 7 mm of space to relieve crowding between the canine and the midline.

The entries into the chart of Figure 13 show the net 7 mm of movement of the teeth between the canine and the midline in the upper right quadrant, and the net 7 mm of movement of the teeth between the canine and the midline in the upper left quadrant.

A computer program 100, which is shown in Figures 14A-14C by way of a flow chart and which is executed by a computer, may be used to implement the present invention. Upon start up of the program 100, a block 102 prompts the user to enter 3x3 crowding as a negative number or 3x3 available spacing as a positive number. Upon entry of this data, a block 104 causes this data to be inserted into the C/S 3x3 row of the arch discrepancy chart (Figure 9). Using the exemplary data of Figures 11-13, the block 104 inserts -5 in the R columns and -1 in the L columns of the 3x3 and 7x7 tables, as shown in Figure 12.

A block 106 prompts the user to enter crowding as a negative number or available spacing as a positive number for the bicuspid regions. Upon entry of this data, a block 108 causes this data to be inserted into the C/S Bicuspid/E row of the arch discrepancy chart. Using the exemplary data of Figures 11-13, the block 108 inserts 1.5 in the R and L columns of the 7x7 table, as shown in Figure 12.

A block 110 prompts the user to enter crowding as a negative number or available spacing as a positive number for the molar regions. Upon entry of this data, a block 112 causes this data to be inserted into the C/S Molars row of the arch discrepancy chart. Using the exemplary data of Figures 11-13, the block 112 inserts -1.5 in the R and L columns of the 7x7 table, as shown in Figure 12.

A block 114 prompts the user to enter crowding as a negative number for any correction of the curve of Spee. Upon entry of this data, a block 116 causes this data to be inserted into the Curve of Spee row of the arch discrepancy chart. Using the exemplary data of Figures 11-13, the block 116 inserts -0.5 in the R and L columns of the 3x3 table and the 7x7 table, as shown in Figure 12.

A block 118 prompts the user to enter crowding as a negative number or available spacing as a positive number resulting from correction of the midline deviation. Upon entry of this data, a block 120 causes this data to be inserted into the Midline row of the arch discrepancy chart. Using the exemplary data of Figures 11-13, the block 120 inserts 2 in the R columns of the 3x3 table and the 7x7 table and inserts -2 in the L columns of the 3x3 table and the 7x7 table, all as shown in Figure 12. The block 120 also inserts the midline deviation into the midline chart (Figure 8). Using the exemplary data of Figures 11-13, the block 120 accordingly inserts 2 for the midline deviation, as shown in Figure 11.

A block 122 prompts the user to enter crowding as a negative number resulting from correction of the incisor positions. Upon entry of this data, a block 124 causes this data to be inserted into the Incisor Position row of the arch discrepancy chart. Using the exemplary data of Figures 11-13, the block 124 inserts -2 in the R and L columns of the 3x3 table and the 7x7 table, as shown in Figure 12.

A block 126 totals (sums) the entries into R and L columns discussed so far and enters these totals as an initial discrepancy into the Initial Discrepancy row of the arch discrepancy chart. Using the exemplary data of Figures 11-13, the block 126 inserts -5.5 in the R and L columns of the 3x3 table and the 7x7 table, as shown in Figure 12.

A block 128 prompts the user to enter space created by stripping, expansion, and distalizing as a positive number, assuming that stripping, expansion, and distalizing are contemplated in the anticipated treatment. Upon entry of this data, a block 130 causes the stripping data to be inserted into the Stripping row of the arch discrepancy chart. Using the exemplary data of Figures 11-13, the block 130 inserts 0 in the R and L columns of the 3x3 table and the 7x7 table, as shown in Figure 12. The block 130 also causes the expansion data to be inserted into the Expansion row of the arch discrepancy chart. Using the exemplary data of Figures 11-13, the block 130 inserts 0 in the R and L columns of the 3x3 table and the 7x7 table, as shown in Figure 12. The block 130 further causes the distalizing data to be inserted into the Distalizing row of the arch discrepancy chart. Using the exemplary data of Figures 11-13, the block 130 inserts 0 in the R and L columns of the 7x7 table, as shown in Figure 12.

A block 132 prompts the user to enter the available space resulting from extractions, assuming that extractions are contemplated in the anticipated treatment. Upon entry of this data, a block 134 causes this data to be inserted into the Extraction row of the arch discrepancy chart. Using the exemplary data of Figures 11-13, the block 124 inserts 7 in the R and L columns of the 7x7 table, as shown in Figure 12.

A block 136 totals (sums) the initial discrepancy with the other space created by stripping, expansion, distalizing, and extraction, and inserts these totals as a remaining discrepancy into the Remaining Discrepancy row of the arch discrepancy chart. Using the exemplary data of Figures 11-13, the block 136 inserts -5.5 in the R and L columns of the 3x3 table and inserts 1.5 in the R and L columns of the 7x7 table, as shown in Figure 12.

A block 138 inserts the anticipated treatment into the chart of Figure 10. Thus, for the lower arch, the block 138 inserts into the chart of Figure 10 the movement of the midline, the space created by extractions (if any), the movement of the teeth in the cuspid to midline region, and the movement of the teeth in the molar region. Using the exemplary data of Figures 11-13, and as shown in Figure 13, the block 138 inserts -2 to show the midline movement, inserts the 5.5 mm distal movement of the teeth in the cuspid to midline regions on the right and left sides, inserts the 3 mm mesial movement of the teeth in the molar regions on the right and left sides, and inserts the 8.5 mm of combined extraction and leeway space.

For the upper arch, the block 138 inserts into the chart of Figure 10 the movement of the midline, the space created by extractions (if any), the movement of the teeth in the cuspid to midline region, and the movement of the teeth in the molar region. Using the exemplary data of Figures 11-13, and as shown in Figure 13, the block 138 inserts 0 to show the upper midline movement, inserts the 7 mm distal movement of the teeth in the cuspid to midline regions on the right and left sides, inserts the 0 mm movement of the teeth in the molar regions on the right and left sides, and inserts the 7 mm of extraction space.

A block 140 prompts the user to enter the molar relationships indicating the deviation between the upper and lower first molars of the patient on the right and left sides. Upon entry of this data, a block 142 causes this data to be inserted into the midline chart. Using the exemplary data of Figures 11-13, the block 142 inserts 3 for the right and left molar relationships, as shown in Figure 11.

A block 144 causes the completed charts to be displayed to the user on a monitor. Alternatively, or additionally, the block 140 causes the completed charts to be printed for the user. The presentation of the information in these charts permits the orthodontist to more effectively treat patients.

Certain modifications of the present invention have been discussed above. Other modifications will occur to those practicing in the art of the present invention. For example, various computer prompts are discussed above in order to prompt a user to enter certain data. Typically, these prompts would be provided by way of suitable screen displays. However, voice prompts or other types of prompts may be used.

Moreover, the block 138, as described above, automatically inserts the anticipated treatment into the chart of Figure 10. However, the block 138 could instead be arranged to prompt the user to manually enter the anticipated treatment into the chart of Figure 10 and/or to edit the anticipated treatment automatically and/or manually entered into the chart of Figure 10.

Accordingly, the description of the present invention is to be construed as illustrative only and is for the purpose of teaching those skilled in the art the best mode of carrying out the invention. The details may be varied substantially without departing from the scope of the invention, and the exclusive use of all modifications which are within the scope of the appended claims is reserved.

## Claims

1. A computer implemented method related to orthodontics comprising:
entering crowding/spacing data in a first table, wherein the crowding/spacing data entered into the first table relates only to a cuspid to midline region of a patient's jaw;
entering crowding/spacing data in a second table, wherein the crowding/spacing data entered into the second table relates to a second molar to midline region of the patient's jaw and includes the crowding/spacing data related to the cuspid to midline region of the patient's jaw; and,
planning an orthodontic treatment based upon the crowding/spacing data entered into the first and second tables.

2. The method of claim 1 further comprising adding midline and molar relationships to a midline chart.

3. The method of claim 1 further comprising adding data related to the planned orthodontic treatment to an anticipated treatment chart.

4. The method of claim 1 further comprising:
adding midline and molar relationships to a midline chart; and,
adding data related to the planned orthodontic treatment to an anticipated treatment chart.

5. The method of claim 1, 2, 3, or 4 further comprising displaying the tables and/or charts.

6. The method of claim 1 further comprising:
summing the crowding/spacing data of the first table to create a first total and entering the first total in the first table as a first discrepancy; and,
summing the crowding/spacing data of the second table to create a second total and entering the second total in the second table as a second discrepancy.

7. The method of claim 1 wherein the crowding/spacing data in the first and second tables includes space required to correct a curve of Spee of the patient's jaw.

8. The method of claim 1 wherein the crowding/spacing data in the first and second tables includes space created and required to move a midline of teeth in the patient's jaw.

9. The method of claim 1 wherein further comprising:
summing the crowding/spacing data of the first table to create a first total and entering the first total in the first table as a first initial discrepancy;
summing the crowding/spacing data of the second table to create a second total and entering the second total in the second table as a second initial discrepancy;
entering other created space in the first and second tables;
summing the first total and the other created space to create a third total and entering the third total in the first table as a first remaining discrepancy; and,
summing the second total and the other created space to create a fourth total and entering the fourth total in the second table as a second remaining discrepancy.

10. The method of claim 6 or 9 wherein the planning of an orthodontic treatment includes entering the respective discrepancies from the first and second tables into a discrepancy chart.

## Patentansprüche

1. Computerimplementiertes Verfahren bezüglich Kieferorthopädie, aufweisend:
Eingeben von Dichte-/Abstandsdaten in eine erste Tabelle, wobei sich die in die erste Tabelle eingegebenen Dichte-/Abstandsdaten lediglich auf einen Eckzahn- bis Mittellinienbereich des Kiefers des Patienten beziehen,
Eingeben von Dichte-Abstandsdaten in eine zweite Tabelle, wobei sich die in die zweite Tabelle eingegebenen Dichte-/Abstandsdaten auf einen zweiten Backenzahn- bis Mittellinienbereich des Kiefers des Patienten beziehen und die Dichte-/Abstandsdaten aufweisen, die sich auf den Eckzahn- bis Mittellinienbereich des Kiefers des Patienten beziehen, und
Planen einer orthodontischen Behandlung auf Basis der in die erste und zweite Tabelle eingegebenen Dichte-/Abstandsdaten.

2. Verfahren nach Anspruch 1, ferner aufweisend Hinzufügen der Mittellinien- und Backenzahnbeziehungen zu einer Mittelliniengrafik.

3. Verfahren nach Anspruch 1, ferner aufweisend Hinzufügen der Daten, die sich auf die geplante orthodontische Behandlung beziehen, zu einer Grafik der voraussichtlichen Behandlung.

4. Verfahren nach Anspruch 1, ferner aufweisend:
Hinzufügen der Mittellinien- und Backenzahnbeziehungen zu einer Mittelliniengrafik, und
Hinzufügen der Daten, die sich auf die geplante orthodontische Behandlung beziehen, zu einer Grafik der voraussichtlichen Behandlung.

5. Verfahren nach Anspruch 1, 2, 3, oder 4, ferner aufweisend Darstellen der Tabellen und/oder Grafiken.

6. Verfahren nach Anspruch 1, ferner aufweisend:
Summieren der Dichte-/Abstandsdaten der ersten Tabelle, um eine erste Summe zu erzeugen, und Eingeben der ersten Summe in die erste Tabelle als eine erste Diskrepanz, und
Summieren der Dichte-/Abstandsdaten der zweiten Tabelle, um eine zweite Summe zu erzeugen, und Eingeben der zweiten Summe in die zweite Tabelle als eine zweite Diskrepanz.

7. Verfahren nach Anspruch 1, wobei die Dichte-/Abstandsdaten in der ersten und zweiten Tabelle einen Platz aufweisen, der zum Korrigieren einer Speeschen Kurve des Kiefers des Patienten erforderlich ist.

8. Verfahren nach Anspruch 1, wobei die Dichte-/Abstandsdaten in der ersten und zweiten Tabelle einen Platz aufweisen, der zum Bewegen einer Mittellinie der Zähne im Kiefer des Patienten erzeugt wird und erforderlich ist.

9. Verfahren nach Anspruch 1, ferner aufweisend:
Summieren der Dichte-/Abstandsdaten der ersten Tabelle, um eine erste Summe zu erzeugen, und Eingeben der ersten Summe in die erste Tabelle als eine erste Anfangsdiskrepanz,
Summieren der Dichte-/Abstandsdaten der zweiten Tabelle, um eine zweite Summe zu erzeugen, und Eingeben der zweiten Summe in die zweite Tabelle als eine zweite Anfangsdiskrepanz,
Eingeben eines anderen erzeugten Platzes in die erste und zweite Tabelle,
Summieren der ersten Summe und des anderen erzeugten Platzes, um eine dritte Summe zu erzeugen, und Eingeben der dritten Summe in die erste Tabelle als erste verbleibende Diskrepanz, und
Summieren der zweiten Summe und des anderen erzeugten Platzes, um eine vierte Summe zu erzeugen, und Eingeben der vierten Summe in die zweite Tabelle als zweite verbleibende Diskrepanz.

10. Verfahren nach Anspruch 6 oder 9, wobei das Planen einer orthodontischen Behandlung das Eingeben der jeweiligen Diskrepanzen aus der ersten und zweiten Tabelle in eine Diskrepanzsgrafik aufweist.

## Revendications

1. Méthode mise en oeuvre par ordinateur, concernant l'orthodontie, comprenant :
l'entrée de données de chevauchement/d'espacement dans un premier tableau, les données de chevauchement/d'espacement entrées dans le premier tableau conernant uniquement une région cuspide à médiane de la mâchoire d'un patient ;
l'entrée de données de chevauchement/d'espacement dans un deuxième tableau, les données de chevauchement/d'espacement entrées dans le deuxième tableau concernant une deuxième région molaire à médiane de la mâchoire du patient et
comportant les données de chevauchement/d'espacement concernant la région cuspide à médiane de la mâchoire du patient ; et
la planification d'un traitement orthodontique sur la base des données de chevauchement/d'espacement entrées dans les premier et deuxième tableaux.

2. Méthode selon la revendication 1, comprenant en outre l'addition de relations médianes et molaires à un graphique de ligne médiane.

3. Méthode selon la revendication 1, comprenant en outre l'addition de données concernant le traitement orthodontique planifié sur un graphique de traitement anticipé.

4. Méthode selon la revendication 1, comprenant en outre :
l'addition de relations médianes et molaires à un graphique de ligne médiane ; et
l'addition de données concernant le traitement orthodontique planifié sur un graphique de traitement anticipé.

5. Méthode selon la revendication 1, 2, 3 ou 4, comprenant en outre l'affichage des tableaux et/ou des graphiques.

6. Méthode selon la revendication 1, comprenant en outre :
la sommation des données de chevauchement/d'espacement du premier tableau pour créer un premier total et l'entrée du premier total dans le premier tableau sous forme de première divergence ; et
la sommation des données de chevauchement/d'espacement du deuxième tableau pour créer un deuxième total et l'entrée du deuxième total dans le deuxième tableau sous forme de deuxième divergence.

7. Méthode selon la revendication 1, dans laquelle les données de chevauchement/d'espacement dans les premier et deuxième tableaux incluent l'espace requis pour corriger une courbe de Spee de la mâchoire du patient.

8. Méthode selon la revendication 1, dans laquelle les données de chevauchement/d'espacement dans les premier et deuxième tableaux incluent l'espace créé et requis pour déplacer une ligne médiane des dents dans la mâchoire du patient.

9. Méthode selon la revendication 1, comprenant en outre :
la sommation des données de chevauchement/d'espacement du premier tableau pour créer un premier total et l'entrée du premier total dans le premier tableau sous forme de première divergence initiale ;
la sommation des données de chevauchement/d'espacement du deuxième tableau pour créer un deuxième total et l'entrée du deuxième total dans le deuxième tableau sous forme de deuxième divergence initiale ;
l'entrée d'un autre espace créé dans les premier et deuxième tableaux ;
la sommation du premier total et de l'autre espace créé pour créer un troisième toal et l'entrée du troisième total dans le premier tableau sous forme de première divergence restante ; et
la sommation du deuxième total et de l'autre espace créé pour créer un quatrième total et l'entrée du quatrième total dans le deuxième tableau sous forme de deuxième divergence restante.

10. Méthode selon la revendication 6 ou 9, dans laquelle la planification d'un traitement orthodontique comporte l'entrée des divergences respectives des premier et deuxième tableaux dans un graphique de divergence.
